# EUROPÄISCHE PATENTSCHRIFT

(11) **EP 3 706 661 B1**
(45) Veröffentlichungstag und Bekanntmachung des Hinweises auf die Patenterteilung: **13.07.2022**
(21) Anmeldenummer: 18756414.1
(22) Anmeldetag: 16.08.2018
(51) Int. Cl.: A61C 7/10

(54) **SCHRAUBGETRIEBE**
SCREW GEAR
ENGRENAGE HÉLICOÏDAL

(30) Priorität: 09.11.2017 AT 4332017
(43) Veröffentlichungstag der Anmeldung: 16.09.2020
(73) Patentinhaber: Dénes, Balázs, József, 1206 Genève (CH)
(72) Erfinder: Dénes, Balázs, József, 1206 Genève (CH)
(74) Vertreter: Torggler & Hofmann Patentanwälte - Rankweil
(86) Internationale Anmeldenummer: PCT/EP2018/072232
(87) Internationale Veröffentlichungsnummer: WO 2019/091614

(56) Entgegenhaltungen:
- CN-U- 204 477 224
- DE-A1- 3 035 119
- DE-C- 952 960
- FR-A- 1 456 555
- GB-A- 250 324
- US-A- 839 821
- US-A- 1 901 286
- US-A1- 2001 036 614
- US-A1- 2010 190 126
- US-A1- 2011 143 300
- US-A1- 2016 120 622

## Beschreibung

Die Erfindung bezieht sich auf ein Schraubgetriebe umfassend eine erste und eine zweite Schraube, die jeweils ein Kopfende und ein gegenüberliegendes Fußende aufweisen, wobei eine jeweilige Schraube im Bereich des Kopfendes mit einem anderen Teil einer das Schraubgetriebe aufweisenden Vorrichtung verbindbar ist, und von denen die erste Schraube einen Gewindeabschnitt mit einem Rechtsgewinde und die zweite Schraube einen Gewindeabschnitt mit einem Linksgewinde aufweist, und eine gemeinsame Mutter, welche eine Durchgangsöffnung aufweist, deren Wandung mit einem ersten Gegengewinde für die erste Schraube und einem zweiten Gegengewinde für die zweite Schraube versehen ist, wobei die erste und zweite Schraube von gegenüberliegenden Seiten der Mutter her in die Gegengewinde der Mutter einschraubbar sind.

Schraubgetriebe, auch als Schraubengetriebe oder Bewegungsschrauben bezeichnet, werden in unterschiedlichen Anwendungsgebieten eingesetzt. Schraubgetriebe dienen zur Umwandlung einer Drehbewegung in eine Längsbewegung. Durch Drehung einer Schraube (=Spindel) wird eine auf der Schraube unverdrehbar gehaltene Mutter in Längsrichtung der Schraube bewegt oder umgekehrt, d.h. durch Drehung der Mutter wird die unverdrehbar gehaltene Schraube in ihre Längsrichtung verschoben.

Bekannt sind Spannschlösser zum Spannen von Drähten und anderen Teilen. Das Spannschloss wird zwischen die zu spannenden Teile eingehängt, wobei eine erste Schraube mit einem Rechtsgewinde mit dem ersten Teil und eine zweite Schraube mit einem Linksgewinde mit dem zweiten Teil verbunden ist. Eine gemeinsame Mutter weist ein erstes Gegengewinde in Form eines Rechtsgewindes für die erste Schraube sowie ein zweites Gegengewinde in Form eines Linksgewindes für die zweite Schraube auf.

Ein Schraubgetriebe der eingangs genannten Art ist speziell im Zusammenhang mit der kieferorthopädischen Anwendung der Gaumennahterweiterung bekannt. Die beiden Schrauben werden mit den beidseitig der Gaumennaht liegenden Teilen des Gaumens verbunden. Hierzu können Miniankerschrauben in den Knochen eingesetzt werden, mit denen die beiden, das Rechts- und das Linksgewinde aufweisenden Schrauben verbunden werden. Eine andere Möglichkeit sieht eine Befestigung an den Zähnen vor. Eine derartige, ein Schraubgetriebe der eingangs genannten Art aufweisende Vorrichtung, die auch als Expandervorrichtung oder Distraktionsvorrichtung bezeichnet wird, geht beispielsweise aus der US 6,450,806 B2 hervor.

Um bei einer platzsparenden Ausbildung einen möglichst großen Hub zu erreichen, sieht eine weitere vorbekannte Expandervorrichtung vor, dass eine der beiden Schrauben mit einem wesentlich größeren Durchmesser als die andere der beiden Schrauben ausgebildet ist und an ihrem der anderen Schraube zugewandten Fußende eine Sacklochausnehmung aufweist, in welche die andere Schraube mit ihrem Fußende eintauchen kann. Das Gegengewinde der Mutter für die Schraube mit dem kleineren Durchmesser ist hierbei entsprechend kürzer ausgebildet. Die mechanischen Eigenschaften für die kleinere Schraube wird durch diese Konstruktion wesentlich verschlechtert.

Die US 2015/0056566 A1 und US 6,499,996 B2 zeigen weitere kieferorthopädische Expandervorrichtungen mit Schraubgetrieben. Das Schraubgetriebe weist jeweils eine Doppelschraube mit in entgegengesetzten Richtungen weisenden Gewindeabschnitten auf, von denen einer mit einem Rechts- und der andere mit einem Links-Gewinde versehen ist. Die Gewindeabschnitte sind jeweils in eine Mutter mit einem entsprechenden Gegengewinde eingeschraubt, wobei die Muttern durch eine Drehung der Doppelschraube auseinander gedrückt werden.

In der CH 709169 A1 wird ein Gaumenspreizer geoffenbart, welcher durch die Kombination von Führungsbuchsen und einer darin versenkbaren Expansionsschraube gegenüber den üblichen Gaumenspreizern einen erweiterten Hub besitzt. Die einzelnen Auslenkungen der drei Teile (Expansionsschrauben und zwei Gewindebuchsen) werden zu einer Gesamtauslenkung addiert, sodass der Hub pro Umdrehung gegenüber typischen Gaumenspreizern bei gleichem Platzbedarf erhöht ist.

Die EP 1 250 100 B1 zeigt eine Vorrichtung zur Erweiterung der Gaumennaht nach vorhergehender Osteotomie. Die Vorrichtung umfasst zwei ineinander versenkbare Schrauben, eine Mutter, welche die Schrauben umgibt und durch Drehung eine Axialverschiebung der Schrauben bewirkt, eine Blockiereinrichtung und mehrere Verankerungs- und Verbindungsteile. Auch bei dieser Vorrichtung ist der Hub gegenüber typischen Gaumenspreizern bei gleichem Platzbedarf durch die Teleskopwirkung vergrößert.

Die US 6,012,920 A offenbart ein Schraubgetriebe zur Anwendung in der Kieferorthopädie, wobei der Relativabstand zwischen dem Ober- und Unterkiefer eingestellt wird. Hierzu umfasst die Vorrichtung eine Hülle in dessen ersten Bereich ein linksgängiges und in dessen zweiten Bereich ein rechtsgängiges Gewinde eingeschnitten wurde. Durch Rotation der Hülle um ihre Längsachse wird der Relativabstand der Schrauben zueinander verstellt, woraufhin sich der Hub der gesamten Vorrichtung entsprechend verändert.

In der US 2010/209212 A1 wird eine Vorrichtung beschrieben die im Bereich ihrer Längsachse einen von ihrem Fußende ausgehenden Schlitz umfasst, der sich über einen Teil der axialen Streckung des Gewindes spannt und den Gewindeabschnitt der Schraube in mehrere Teilabschnitte trennt, deren Außenflächen Umfangsteile von Kreiszylinder-Mantelflächen bilden. Ferner umfasst die Vorrichtung auch eine Mutter mit einem zum Gewinde der Schraube passenden Gegengewinde, welche dazu ausgebildet ist die Schraube und einen zum geteilten Schraubenschaft komplementären Abschnitt aufzunehmen.

GB 250 324 A offenbart eine Schraubgetriebe nach dem Oberbegriff von Anspruch 1.

Aufgabe der Erfindung ist es ein vorteilhaftes Schraubgetriebe der eingangs genannten Art bereitzustellen, wobei eine kompaktere Bauweise und/oder ein größerer Hub erreicht wird. Erfindungsgemäß gelingt dies durch ein Schraubgetriebe mit den Merkmalen des Anspruchs 1.

Beim Schraubgetriebe der Erfindung weisen die Gewindeabschnitte der ersten und zweiten Schraube jeweils mindestens zwei Teilabschnitte auf. Die Außenflächen dieser Teilabschnitte bilden Umfangsteile von Kreiszylinder-Mantelflächen, welche bei der ersten Schraube mit einem Rechts- und bei der zweiten Schraube mit einem Linksgewinde versehen sind. Mindestens eine der Schrauben weist zwischen den Teilabschnitten des Gewindeabschnitts der Schraube einen Spalt auf. Dieser Spalt erstreckt sich im Bereich der Längsmittelachse der Schraube und geht vom Fußende der Schraube aus. Durch diese Ausbildung sind die erste und zweite Schraube in einer Ausrichtung, in welcher ihre Fußenden zueinander gerichtet sind, axial ineinander einsteckbar. Vorteilhafterweise sind die beiden Schrauben zumindest so weit axial ineinander einsteckbar, dass die Gewindeabschnitte der Schrauben über mindestens die Hälfte ihrer axialen Ausdehnungen (=ihrer Erstreckungen parallel zur Längsmittelachse der jeweiligen Schraube) überlappen, vorzugsweise über mindestens zwei Drittel ihrer axialen Ausdehnungen, besonders bevorzugt über ihre gesamten axialen Ausdehnungen.

Die ersten und zweiten Gegengewinde der Mutter sind zumindest über einen Teil ihrer axialen Ausdehnungen ineinander liegend in den gleichen axialen Abschnitt der Wandung der Durchgangsöffnung der Mutter eingebracht. Vorteilhafterweise ist dies zumindest über 50% der axialen Ausdehnungen der ersten und zweiten Gegengewinde der Fall, vorzugsweise über zumindest zwei Drittel. In einer möglichen Ausführung können die ersten und zweiten Gegengewinde über ihre gesamten axialen Erstreckungen ineinander liegend in die Wandung der Durchgangsöffnung eingebracht sein. Die Wandung der Durchgangsöffnung ist damit vom einen bis zum anderen Ende durchgehend sowohl mit einem Rechts- als auch mit einem Linksgewinde versehen.

Ein erfindungsgemäßes Schraubgetriebe zeichnet sich durch einen großen Hub bei einer sehr kompakten Bauweise aus und weist dabei eine gute Stabilität auf.

Ein erfindungsgemäßes Schraubgetriebe ist vorteilhaft in der Kieferorthopädie bzw. Orthodontie einsetzbar, insbesondere in einer Vorrichtung zur Erweiterung der Gaumennaht.

Günstigerweise ergänzen sich die Teilabschnitte der ersten und zweiten ineinander eingesteckten Schrauben im Bereich der axialen Überlappung der Teilabschnitte der ersten und zweiten Schrauben zu einem vollständigen Kreiszylinder. Es wird dadurch eine möglichst große Stabilität erreicht.

Eine vorteilhafte Ausführungsform sieht vor, dass der Gewindeabschnitt einer jeweiligen Schraube genau zwei Teilabschnitte aufweist. Die Außenflächen dieser Teilabschnitte, welche die mit den Gewinden versehenen Umfangsteile von Kreiszylinder-Mantelflächen bilden, erstrecken sich hierbei günstigerweise jeweils über 80° bis 100°. Eine Ausbildung der Schrauben mit jeweils drei oder mehr Teilabschnitten ist aber denkbar und möglich.

Die Erfindung bezieht sich im Weiteren auf eine kieferorthopädische Expandervorrichtung zur Gaumennahterweiterung, welche ein erfindungsgemäßes Schraubgetriebe aufweist. Eine solche Expandervorrichtung wird auch als Expander, Distraktionsvorrichtung oder Distraktor bezeichnet. Zur transversalen Erweiterung des Oberkiefers wird eine Expansionskraft zwischen den beidseitig der Gaumennaht liegenden Hälften des Oberkiefers erzeugt. Zur Verankerung der Vorrichtung am jeweiligen Gaumenteil sind Verankerungsteile vorgesehen, diese können in einer vorteilhaften Ausführungsform von Ankerschrauben, auch als Miniankerschrauben bezeichnet, gebildet werden, welche im Knochen verankerbar sind. Man spricht in diesem Fall von einer knochengetragenen Expandervorrichtung. Eine andere Möglichkeit besteht darin, Verankerungsteile zur Verankerung der Vorrichtung an den Backenzähnen vorzusehen. Auch eine kombinierte Verankerung, also sowohl mit Verankerungsteilen zur Verankerung im Knochen als auch mit Verankerungsteilen zur Verankerung an den Zähnen ist denkbar und möglich. Im Weiteren weist die Vorrichtung Verbindungsteile zur Verbindung der Schrauben des Schraubgetriebes mit den Verankerungsteilen auf.

Weitere Vorteile und Einzelheiten der Erfindung werden im Folgenden anhand der beiliegenden Zeichnung erläutert. In dieser zeigen:
Fig. 1 eine Schrägsicht eines Schraubgetriebes gemäß der Erfindung, im vollständig zusammengefahrenen Zustand;
Fig. 2 die Schrauben in Seitenansicht und die Mutter im Längsmittelschnitt, im Zustand von Fig. 1;
Fig. 3 einen Querschnitt entlang der Linie AA von Fig. 2;
Fig. 4 und 5 Darstellungen entsprechend Fig. 1 und 2 in einem teilweise auseinandergefahrenen Zustand des Schraubgetriebes;
Fig. 6 eine Darstellung entsprechend Fig. 4, wobei aber die Mutter aufgeschnitten dargestellt ist;
Fig. 7 und 8 Darstellungen entsprechend Fig. 1 und 2 in einem gegenüber den Fig. 4-6 weiter auseinandergefahrenen Zustand des Schraubgetriebes;
Fig. 9 und 10 Darstellungen entsprechend Fig. 1 und 2 im noch weiter auseinandergefahrenen Zustand des Schraubgetriebes;
Fig. 11, 12 und 13 die erste Schraube des Schraubgetriebes in einer Schrägsicht, Seitenansicht und stirnseitigen Ansicht (auf das Fußende der Schraube);
Fig. 14, 15 und 16 Darstellungen entsprechend Fig. 11, 12 und 13 für die zweite Schraube;
Fig. 17 und 18 eine Schrägsicht und eine stirnseitige Ansicht der Mutter;
Fig. 19 einen Schnitt entlang der Linie CC von Fig. 18;
Fig. 20 einen Schnitt entlang der Linie BB von Fig. 18;
Fig. 21 und 22 eine Schrägsicht der ersten und zweiten Schraube eines zweiten Ausführungsbeispiels eines Schraubengetriebes gemäß der Erfindung;
Fig. 23 eine Schrägsicht des Schraubengetriebes gemäß einem dritten Ausführungsbeispiel der Erfindung;
Fig. 24 eine Darstellung entsprechend Fig. 23, ohne die Mutter und die Halteklammern;
Fig. 25 und 26 eine Schrägsicht und eine Seitenansicht der ersten Schraube des Schraubengetriebes gemäß einer vierten Ausführungsform der Erfindung;
Fig. 27 eine stirnseitige Ansicht auf das Fußende der ersten Schraube;
Fig. 28 und 29 eine Schrägsicht und eine Seitenansicht der zweiten Schraube des Schraubengetriebes gemäß der vierten Ausführungsform der Erfindung;
Fig. 30 eine stirnseitige Ansicht auf das Fußende der zweiten Schraube;
Fig. 31 und 32 vereinfachte Darstellungen des Einsatzes eines erfindungsgemäßen Schraubengetriebes in einer Expandervorrichtung zur Gaumennahterweiterung (Fig. 31 zeigt eine intraorale Ansicht, Fig. 32 einen koronalen Schnitt);
Fig. 33 eine schematische Darstellung einer Hilfsvorrichtung zur Montage des Schraubengetriebes.

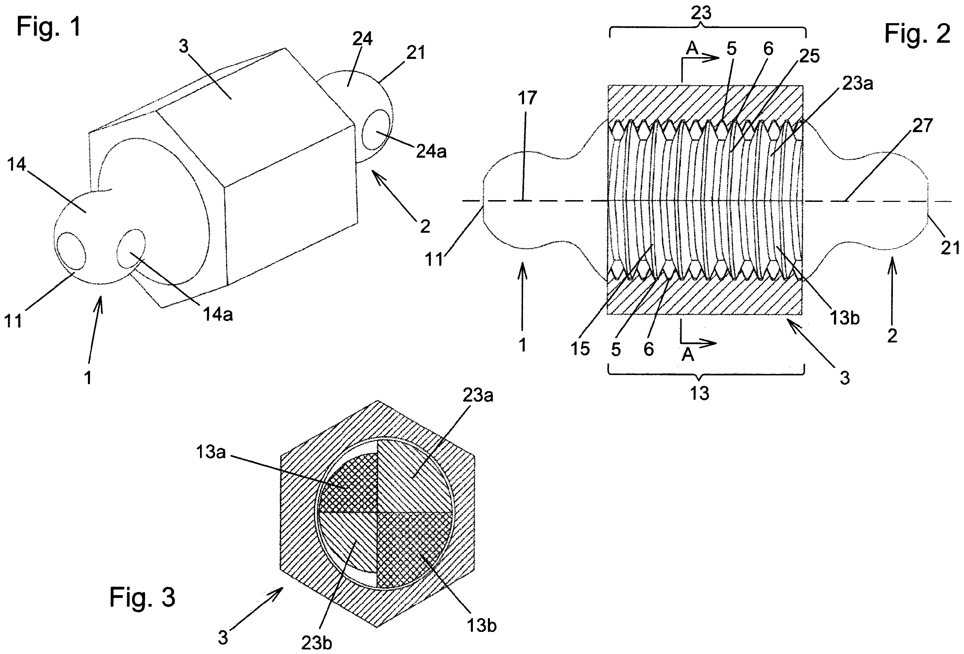

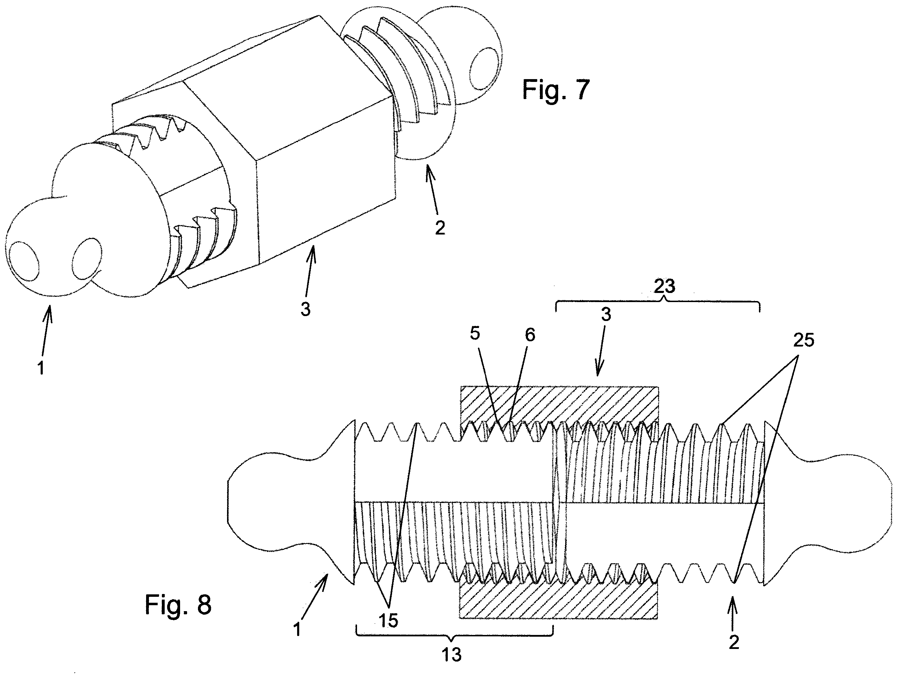

Ein erstes Ausführungsbeispiel der Erfindung wird im Folgenden anhand der Fig. 1-22 erläutert. Das Schraubgetriebe, das auch als Schraubvorrichtung bezeichnet werden kann, weist eine erste Schraube 1, eine zweite Schraube 2 und eine gemeinsame Mutter 3 auf.

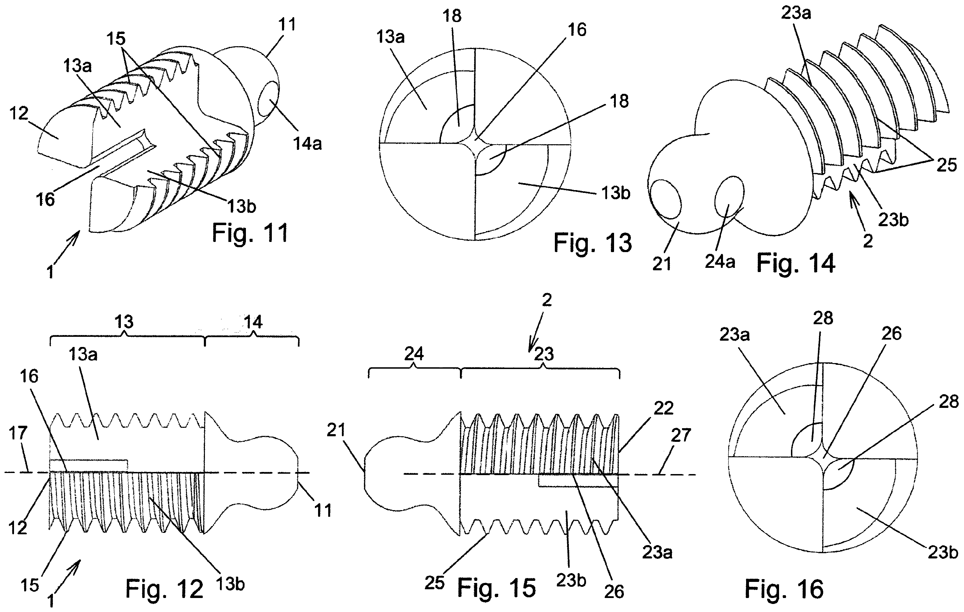

Die Ausbildung der Schrauben 1, 2 ist am besten aus den Fig. 11-16 ersichtlich. Die Schrauben 1, 2 besitzen jeweils ein Kopfende 11, 21, im Bereich von dem die jeweilige Schraube mit einem anderen Teil einer das Schraubgetriebe aufweisenden Vorrichtung verbindbar ist, und ein gegenüberliegendes Fußende 12, 22. Das Kopfende 11, 21 liegt endseitig an einem Kopfabschnitt 14, 24 der jeweiligen Schraube 1, 2. Am gegenüberliegenden Ende ist der Kopfabschnitt 14, 24 mit einem Gewindeabschnitt 13, 23 der jeweiligen Schraube verbunden. Der Gewindeabschnitt 13, 23 schließt in axialer Richtung der Schraube, also in Richtung der Längserstreckung der Schraube, an den Kopfabschnitt 14, 24 der jeweiligen Schraube an. Das vom Kopfabschnitt 14 abgelegene Ende des Gewindeabschnitts 13, 23 bildet das Fußende 12, 22 der jeweiligen Schraube 1, 2.

Der Kopfabschnitt 14, 24 der jeweiligen Schraube 1, 2 besitzt ein Verbindungsmittel 14a, 24a, um den Kopfabschnitt 14, 24 mit dem jeweiligen anderen Teil der das Schraubgetriebe aufweisenden Vorrichtung zu verbinden. Das Verbindungsmittel 14a, 24a wird im Ausführungsbeispiel von der Form des Kopfabschnittes mit gegenüberliegenden Abflachungen gebildet, könnte beispielsweise aber auch von einer querliegenden Durchgangsöffnung durch den Kopfabschnitt 14, 24 gebildet werden.

Der Gewindeabschnitt 13, 23 der jeweiligen Schraube 1, 2 weist zwei Teilabschnitte 13a, 13b; 23a, 23b auf, wobei die Außenflächen dieser Teilabschnitte Umfangsteile von Kreiszylinder-Mantelflächen darstellen, welche bei der ersten Schraube 1 mit dem Rechtsgewinde 15 und bei der zweiten Schraube 2 mit dem Linksgewinde 25 versehen sind. Das jeweilige Gewinde 15, 25 ist also unvollständig, indem es sich nur über die Teile des gesamten Umfangs erstreckt, über welche die Teilabschnitte 13a, 13b; 23a, 23b verlaufen und dazwischen unterbrochen ist. Die Teilabschnitte 13a, 13b; 23a, 23b stellen also jeweils im Wesentlichen Zylindersektoren dar (welche an ihren Außenflächen mit Gewinden versehen sind und im Bereich der zentralen Längsachse Aussparungen aufweisen, welche die weiter unten genauer beschriebenen Schlitze bilden).

Im Ausführungsbeispiel erstrecken sich die mit dem jeweiligen Gewinde 15, 25 versehenen Außenflächen der Teilabschnitte 13a, 13b; 23a, 23b jeweils über einen Winkel 18, 28 von 90° um die Längsmittelachse 17, 27. Die Teilabschnitte 13a, 13b; 23a, 23b stellen also jeweils im Wesentlichen Viertelzylinder dar.

Zwischen den beiden Umfangsteilen von Kreiszylinder-Mantelflächen, die von den beiden Teilabschnitten 13a, 13b; 23a, 23b gebildet werden, liegen Lücken, welche im Ausführungsbeispiel jeweils 90° betragen.

Eine jeweilige Schraube 1, 2 weist einen im Bereich der zentralen Längsmittelachse 17, 27 der jeweiligen Schraube 1, 2 liegenden Schlitz 16, 26 auf, der die beiden Teilabschnitte 13a, 13b; 23a, 23b voneinander trennt. Dieser Schlitz 16, 26 geht vom Fußende 12, 22 der Schraube 1, 2 aus und verläuft in axialer Richtung der Schraube 1, 2 über zumindest einen Teil des Gewindeabschnitts 13, 23. Im Ausführungsbeispiel verlaufen die Schlitze 16, 26 beider Schrauben 1, 2 jeweils über die Hälfte der Längserstreckungen (=axiale Ausdehnungen) der Gewindeabschnitte 13, 23.

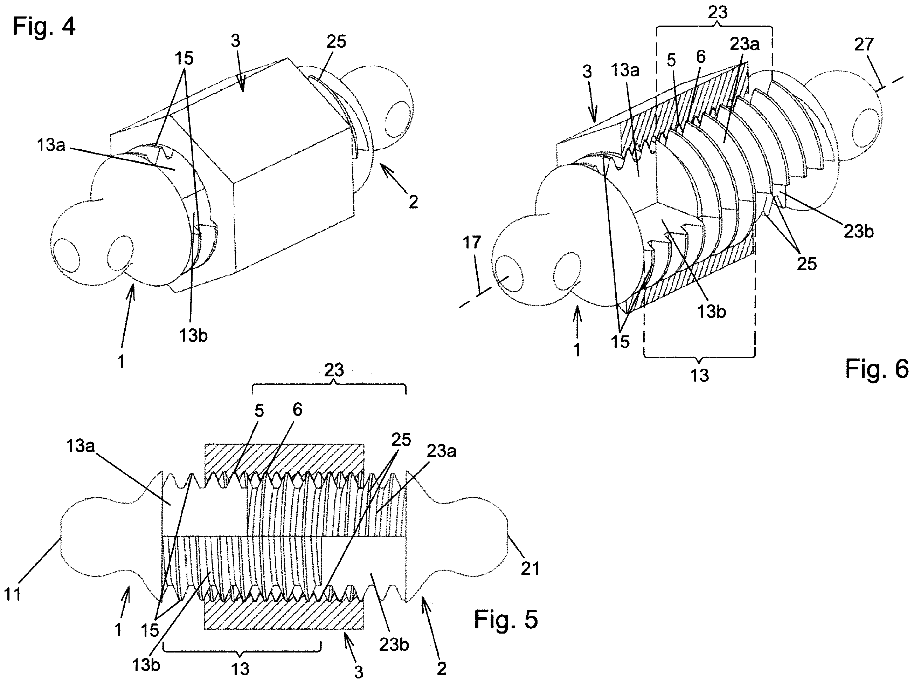

Die koaxial angeordneten und mit ihren Fußenden 12, 22 zueinander gerichteten Schrauben 1, 2, die bezogen auf die Drehrichtung um die Längsmittelachse 17, 27 so ausgerichtet sind, dass die Teilabschnitte 13a, 13b der ersten Schraube den Lücken zwischen den Teilabschnitten 23a, 23b der zweiten Schraube 2 gegenüberliegen und umgekehrt, können axial ineinander eingesteckt werden, vgl. Fig. 6. Vorzugsweise können die beiden Schrauben 1, 2 hierbei so weit ineinander eingesteckt werden, dass ihre Gewindeabschnitte 13, 23 über mehr als die Hälfte der Längserstreckungen der Gewindeabschnitte 13, 23, besonders bevorzugt im Wesentlichen über die gesamten Längserstreckungen der Gewindeabschnitte 13, 23 einander überlappen.

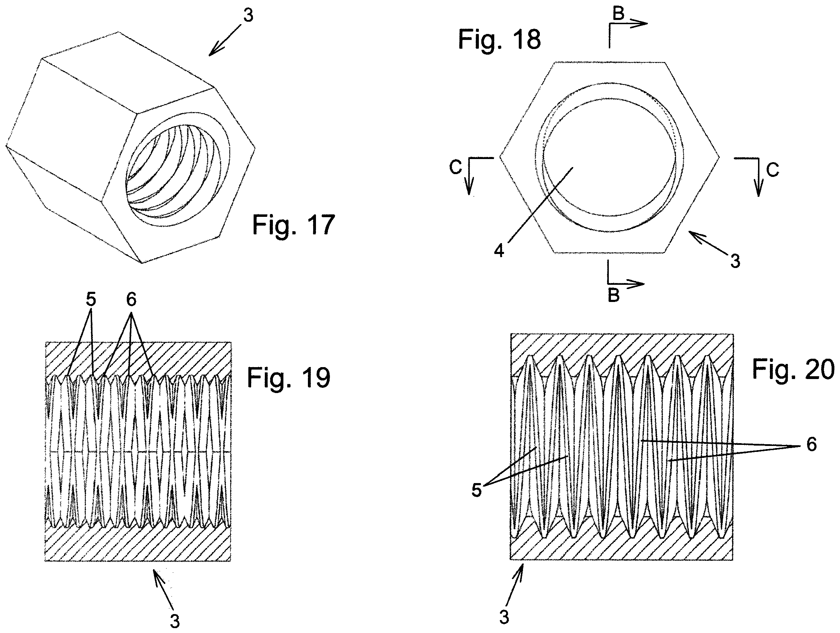

Die Ausbildung der gemeinsamen Mutter 3 für die Schrauben 1, 2 ist am besten aus Fig. 17-20 ersichtlich. Die Mutter 3 besitzt eine axiale Durchgangsöffnung 4. Deren Wandung ist mit einem ersten Gegengewinde 5 für das Rechtsgewinde 15 des Gewindeabschnitts 13 der ersten Schraube 1 und mit einem zweiten Gegengewinde 6 für das Linksgewinde 25 des Gewindeabschnitts 23 der zweiten Schraube 2 versehen. Das erste und das zweite Gegengewinde 5, 6 sind hierbei im Ausführungsbeispiel über ihre gesamten axialen Erstreckungen ineinander liegend (=überlappend) in die Wandung der Durchgangsöffnung 4 eingebracht. Es erstreckt sich also sowohl das erste Gegengewinde 5 als auch das zweite Gegengewinde 6 über die gesamte Längserstreckung (=axiale Ausdehnung) der Durchgangsöffnung 4, also von einem Ende der Durchgangsöffnung 4 bis zum anderen Ende. Die Wandung der Durchgangsöffnung 4 weist also die Form eines Kreiszylindermantels auf, der mit den ineinander liegenden Gegengewinden 5, 6 versehen ist.

Die beiden Gegengewinde 5, 6 können in die Durchgangsöffnung 4 der Mutter eingebracht werden, indem zuerst das erste Gegengewinde 5 mit einem Gewindeschneider für ein Rechtsgewindes und dann das zweite Gegengewinde 6 mit einem Gewindeschneider für ein Linksgewinde eingebracht wird.

Zur Montage des Schraubgetriebes wird die erste Schraube 1 mit ihrem Fußende 12 am einen Ende der Durchgangsöffnung 4 der Mutter 3 angesetzt und die zweite Schraube 2 wird mit ihrem Fußende 22 am anderen Ende der Durchgangsöffnung 4 der Mutter 3 angesetzt, und zwar jeweils derart, dass das Rechtsgewinde 15 des Gewindeabschnitts 13 der ersten Schraube 1 in den Beginn des ersten Gegengewindes 5 eingreift und das Linksgewinde 25 des Gewindeabschnitts 23 der zweiten Schraube 2 in den Beginn des zweiten Gegengewindes 6 eingreift. In der Folge wird die Mutter 3 gedreht, sodass durch den Gewindeeingriff des Rechtsgewindes 15 in das erste Gegengewinde 5 und des Linksgewindes 25 in das zweite Gegengewinde 6 die Schrauben 1, 2 zueinander gezogen werden. Die Schrauben 1, 2 werden hierbei bezogen auf die Drehrichtung um die zusammenfallenden Längsmittelachsen 17, 27 so ausgerichtet, dass die Teilabschnitte 13a, 13b des Gewindeabschnitts 13 der ersten Schraube 1 den Lücken zwischen den Teilabschnitten 23a, 23b des Gewindeabschnitts 23 der zweiten Schraube 2 gegenüberliegen und umgekehrt. Die erste und zweite Schraube 1, 2 können daher durch die Drehung der Mutter 3 nicht nur so weit in Richtung axial zueinander verschoben werden, dass ihre Fußenden 12, 22 an der gleichen axialen Stelle zu liegen kommen, sondern vielmehr durch eine weitere Drehung der Mutter 3 ineinander eingeschoben werden, vgl. Fig. 4-6, bis der vollständig zusammengeschobene Zustand erreicht wird, vgl. Fig. 1-3.

Im vollständig zusammengeschobenen Zustand überlappen die Gewindeabschnitte 13, 23 im Ausführungsbeispiel über ihre gesamten axialen Erstreckungen.

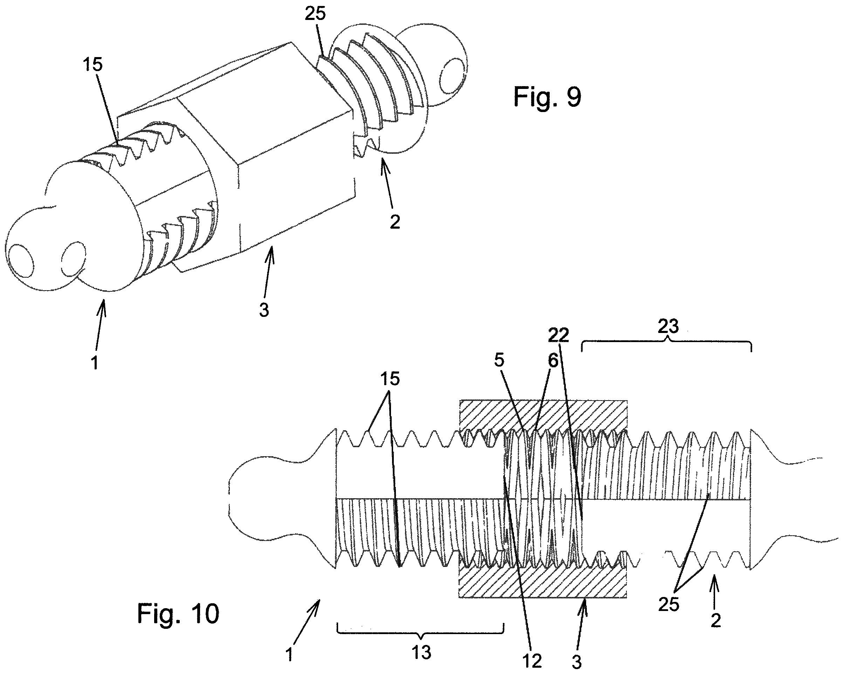

Durch eine entgegengesetzte Drehung der Mutter können die Schrauben 1, 2 wieder zunehmend auseinander geschoben werden. Fig. 9 und 10 zeigen einen empfohlenen maximal auseinander geschobenen Zustand, in welchem noch eine ausreichend stabile Verbindung zwischen den Schrauben 1, 2 und der Mutter 3 vorliegt. Im maximal auseinandergeschobenen Zustand überlappen die Gewindeabschnitte 13, 23 der Schrauben 1, 2 bezogen auf die axiale Richtung (=die Richtung parallel zu den Längsachsen 17, 27) im Ausführungsbeispiel nicht.

Zwischen dem in den Fig. 1-3 dargestellten Zustand (=vollständig zusammen geschobener Zustand) und dem in den Fig. 9, 10 dargestellten Zustand (=maximal auseinander geschobener Zustand) liegt der Arbeitsbereich des Schraubgetriebes. Im Ausführungsbeispiel überlappen die Gewindeabschnitte 13, 23 der Schrauben 1, 2 bezogen auf die axiale Richtung über etwa die Hälfte des Expansionsweges des Schraubgetriebes.

Um ausgehend vom in den Fig. 9 und 10 dargestellten Zustand ein weiteres Auseinanderfahren der Schrauben 1, 2 zu verhindern (sodass diese aus den Gegengewinden 5, 6 der Mutter 3 hinausbewegt werden), können entsprechende Anschläge an der Vorrichtung vorgesehen sein, in welche das Schraubgetriebe eingebaut wird. Grundsätzlich denkbar und möglich wäre es auch, am Fußende 12, 22 zumindest einer der Schrauben 1, 2 einen Fortsatz mit einem Anschlagelement vorzusehen, der im vollständig auseinandergefahrenen Zustand an einen Gegenanschlag der anderen Schraube anläuft.

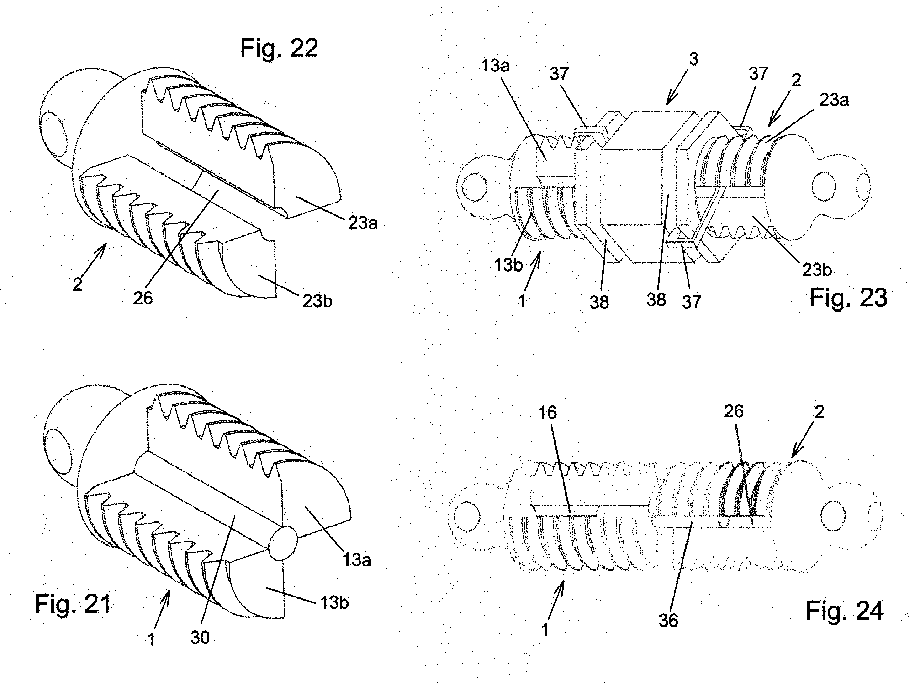

Ein zweites Ausführungsbeispiel der Erfindung wird im Folgenden anhand der Fig. 21 und 22 erläutert. Abgesehen von den im Folgenden beschriebenen Unterschieden entspricht die Ausbildung derjenigen des ersten Ausführungsbeispiels und die Beschreibung des ersten Ausführungsbeispiels ist insoweit analog heranziehbar.

In diesem zweiten Ausführungsbeispiel der Erfindung weist nur eine der beiden Schrauben, beispielsweise die zweite Schraube 2 einen Schlitz 26 auf, der sich, ausgehend vom Fußende 22 der Schraube in axialer Richtung im Bereich der Längsmittelachse 27 erstreckt und die Teilabschnitte 23a, 23b trennt. Dieser Schlitz 26 erstreckt sich hierbei günstigerweise über mehr als zwei Drittel der axialen Erstreckung des Gewindeabschnitts 23, beispielsweise wie dargestellt über die gesamte axiale Erstreckung des Gewindeabschnitts 23, um ein entsprechend weites Ineinanderschieben der ersten und zweiten Schraube 1, 2 zu ermöglichen.

Bei der anderen Schraube 1 sind die Teilabschnitte 13a, 13b über ihre gesamte axiale Erstreckung miteinander verbunden, beispielsweise mittels eines im Bereich der zentralen Längsmittelachse 17 der Schraube 1 liegenden stiftförmigen Verbindungsabschnitts 30.

Ein drittes Ausführungsbeispiel ist in den Fig. 23 und 24 dargestellt. Abgesehen von den im Folgenden beschriebenen Unterschieden entspricht die Ausbildung derjenigen des ersten Ausführungsbeispiels und die Beschreibung des ersten Ausführungsbeispiels ist insoweit analog heranziehbar.

In diesem dritten Ausführungsbeispiel der Erfindung weisen beide Schrauben einen Schlitz 16, 26 auf, der sich im Bereich der Längsmittelachse 17, 27 der jeweiligen Schraube ausgehend vom Fußende 12, 22 der Schraube über den gesamten Gewindeabschnitt 13, 23 erstreckt. Die den Schlitz 16, 26 begrenzenden Wandung der Teilabschnitte 13a, 13b; 23a, 23b verlaufen zylindermantelförmig. Im von den Schlitzen 16, 26 gebildeten Hohlraum ist ein Stift 36 angeordnet. An diesem sind Halteelemente 37 angebracht, welche aus den Lücken zwischen den Teilabschnitten 13a, 13b bzw. 23a, 23b herausragen und mit hakenförmig umgebogenen Enden in ringförmigen Nuten 38 an der Außenseite der Mutter 3 eingreifen. Der Stift 36 wird dadurch in einer Position gehalten, in welcher er sich sowohl in den Schlitz 16 als auch in den Schlitz 26 erstreckt, und zwar auch dann wenn die Schrauben 1, 2 so weit auseinandergefahren sind, dass deren Gewindeabschnitte 13, 23 sich nicht mehr axial überlappen (vgl. Fig. 24). Die Stabilität der Gewindeabschnitte wird dadurch erhöht.

Die Schlitze 16, 26 müssten sich in diesem Ausführungsbeispiel nicht vollständig über die gesamten axialen Ausdehnungen der Gewindeabschnitte 13, 23 erstrecken und der Stift 36 könnte auch kürzer ausgebildet sein. Anstelle der Halteelemente 37 und Nuten 38 könnten beispielsweise auch Schraubenfedern zwischen dem Stift 36 und dem Ende des jeweiligen Schlitzes 16, 26 angeordnet sein.

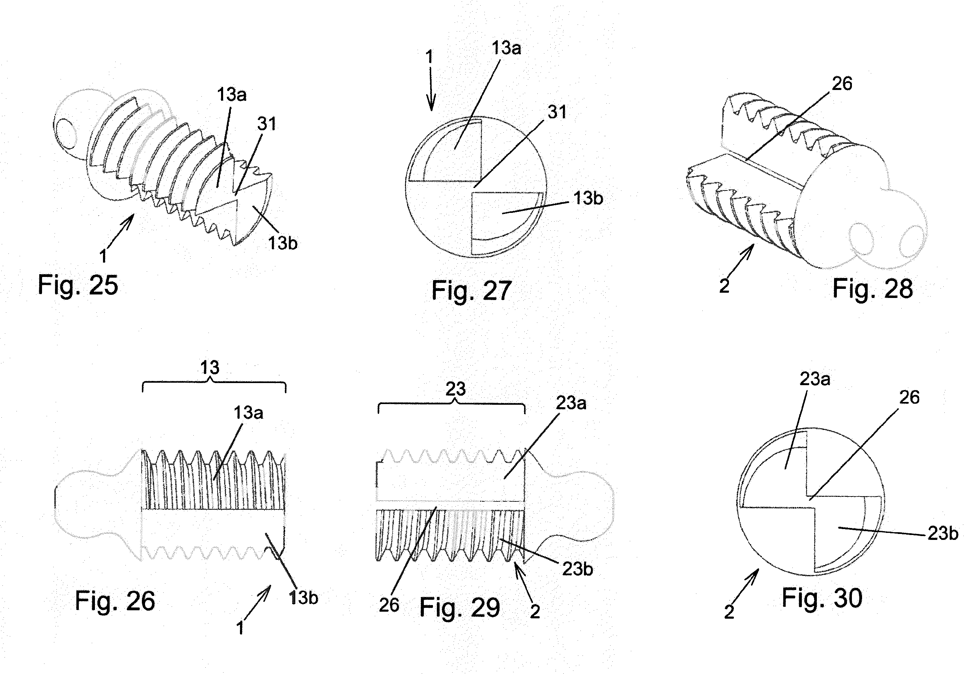

Im Folgenden wird anhand der Fig. 25-30 ein viertes Ausführungsbeispiel der Erfindung erläutert. Abgesehen von den im Folgenden beschriebenen Unterschieden entspricht die Ausbildung derjenigen des ersten Ausführungsbeispiels und die Beschreibung des ersten Ausführungsbeispiels ist insoweit analog heranziehbar.

Beim vierten Ausführungsbeispiel der Erfindung weist wie beim zweiten Ausführungsbeispiel nur eine der beiden Schrauben, beispielsweise die zweite Schraube 2 einen die beiden Teilabschnitte 23a, 23b im Bereich der Längsmittelachse trennenden Schlitz 26 auf, während die Teilabschnitte 13a, 13b des Gewindeabschnitts 13 der anderen Schraube 1 über ihre gesamte axiale Erstreckung verbunden sind. Die Form der Verbindung ist hier aber eine andere als beim zweiten Ausführungsbeispiel, nämlich nach Art eines plattenförmigen Steges 31.

Der Schlitz 26 zwischen den Teilabschnitten 23a, 23b der Schraube 2 ist so ausgebildet, dass der plattenförmige Steg 31 zwischen den Teilabschnitten 13a, 13b der anderen Schraube 1 durch diesen hindurchgeführt werden kann.

Ziel dieser asymmetrischen Teilung der Gewindeabschnitte ist eine bessere Kraftaufnahme von vertikalen Kräften (vertikal bezogen auf Fig. 30), da sich die beiden Gewindeabschnitte bei fehlender Unterstützung des Vollzylinders (bei fehlender Überlappung) verbiegen/verformen können.

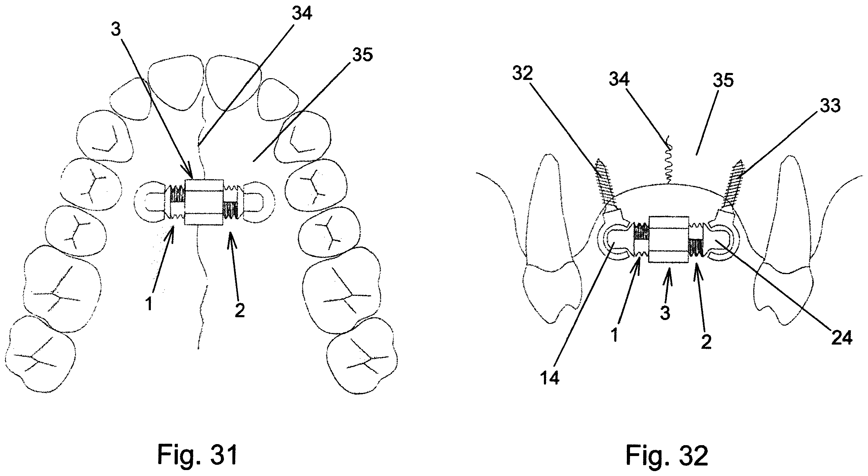

Fig. 31 und 32 zeigen in schematischer Darstellung eine mögliche Anwendung eines erfindungsgemäßen Schraubgetriebes im Bereich der Kieferorthopädie, und zwar in einer Expandervorrichtung zur Gaumennahterweiterung. Eine solche wird auch als Expander, Distraktionsvorrichtung oder Distraktor bezeichnet. Die Kopfabschnitte 14, 24 der beiden Schrauben 1, 2 sind mit Verankerungsteilen 32, 33 verbunden, über welche die Vorrichtung beidseits der Gaumennaht 34 mit dem Gaumen 35 verbunden wird. Bei den Verankerungsteilen 32, 33 handelt es sich um Miniankerschrauben. Die Expandervorrichtung ist in diesem Ausführungsbeispiel also knochengetragen. Auch eine Verankerung an den Backenzähnen oder eine kombinierte Verankerung im Knochen und an den Backenzähnen ist über entsprechende Verbindungs- und Verankerungsteile denkbar und möglich.

In der schematischen Darstellung von Fig. 31 und 32 sind die Kopfabschnitte 14, 24 der Schrauben 1, 2 direkt mit den Verankerungsteilen 32, 33 verbunden. Es könnten auch separate Koppelteile vorhanden sein, welche einerseits mit den Kopfabschnitten 14, 24 der Schrauben 1, 2 und andererseits mit den Verankerungsteilen 32, 33 verbunden sind.

Zu Beginn der Behandlung befindet sich das Schraubgetriebe in einem weitgehend zusammengefahrenen Zustand. Im Laufe der Behandlung, welche sich über mehrere Monate erstrecken kann, werden die Schrauben 1, 2 mittels der Mutter 3 nach und nach auseinandergefahren.

Durch die Erfindung wird bei einer sehr kompakten Ausbildung ein großer Hub ermöglicht.

Unterschiedliche Modifikationen der gezeigten Ausführungsbeispiele sind denkbar und möglich, ohne den Bereich der Erfindung zu verlassen.

So könnte sich beispielsweise das erste Gegengewinde 5 ausgehend von einem ersten Ende der Durchgangsöffnung 4 nicht vollständig bis zum gegenüberliegenden zweiten Ende der Durchgangsöffnung 4 erstrecken, sondern in einem gewissen Abstand davor enden. Umgekehrt könnte sich das zweite Gegengewinde 6 ausgehend vom zweiten Ende der Durchgangsöffnung 4 nicht vollständig bis zum ersten Ende der Durchgangsöffnung 4 erstrecken, sondern in einem gewissen Abstand davor enden. Die Gegengewinde 5, 6 liegen bei dieser Ausführungsform also nur teilweise ineinander (=überlappen nur teilweise). Das Einschrauben der ersten und zweiten Schraube 1, 2 in die Gegengewinde 5, 6 ausgehend vom getrennten Zustand der ersten und zweiten Schraube 1, 2 könnte dadurch beim Zusammensetzen des Schraubgetriebes erleichtert werden.

Der Gewindeabschnitt 13, 23 einer jeweiligen Schraube 1, 2 könnte auch mehr als zwei Teilabschnitte 13a, 13b; 23a, 23b aufweisen, deren Außenflächen jeweils einen Umfangsteil einer Kreiszylinder-Mantelfläche bilden, die mit dem Rechts- bzw. Linksgewinde versehen ist. Beispielsweise könnte ein jeweiliger Gewindeabschnitt 13, 23 drei Teilabschnitte aufweisen, deren Außenflächen sich jeweils über 60° um die Längsmittelachse erstrecken und die jeweils um 60° voneinander beabstandet sind.

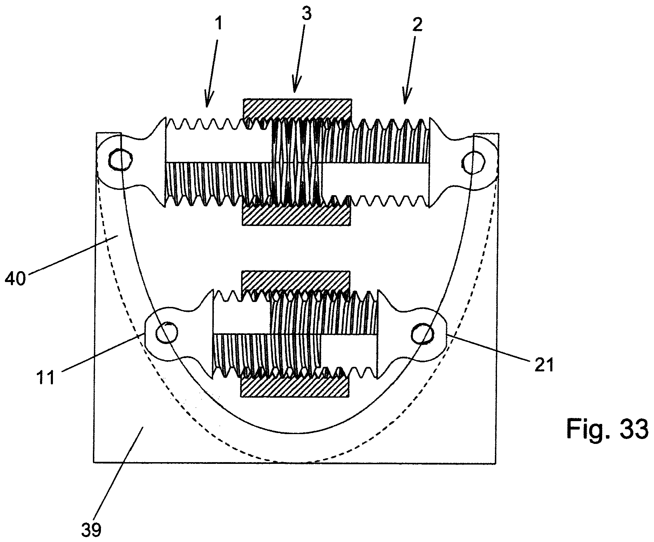

Eine Hilfsvorrichtung (Schablone) zur Erleichterung der Montage des Schraubgetriebes ist schematisch in Fig. 33 dargestellt, Durch diese Hilfsvorrichtung 39 werden die beiden Schrauben 1, 2 in korrekter Position gehalten, während sie in die Mutter 3 eingeschraubt werden. Die Hilfsvorrichtung weist eine bogenförmig begrenzte Ausnehmung mit gegenüberliegenden Führungsrillenabschnitten 40, 41 auf. Die Kopfabschnitte 14, 24 der Schrauben 1, 2 gleiten dabei entlang dieser Führungsrillenabschnitte 40, 41 kontinuierlich entlang und sind durch beidseitige Abflachungen gegen ein Verdrehen gesichert, wobei durch das Drehen der Mutter 3 die beiden Schrauben 1, 2 zusammengezogen werden. Die gegenüberliegenden Führungsrillenabschnitte 40, 41, welche die Kopfabschnitte 14, 24 verdrehgesichert gleitend führen, nähern sich in eine Richtung rechtwinkelig zur Längserstreckung der Schrauben 1, 2 aneinander an (im Ausführungsbeispiel bogenförmig gekrümmt).

### Legende zu den Hinweisziffern:

- 1: erste Schraube
- 2: zweite Schraube
- 3: Mutter
- 4: Durchgangsöffnung
- 5: erstes Gegengewinde
- 6: zweites Gegengewinde
- 11: Kopfende
- 12: Fußende
- 13: Gewindeabschnitt
- 13a: Teilabschnitt
- 13b: Teilabschnitt
- 14: Kopfabschnitt
- 14a: Verbindungsmittel
- 15: Rechtsgewinde
- 16: Schlitz
- 17: Längsmittelachse
- 18: Winkel
- 21: Kopfende
- 22: Fußende
- 23: Gewindeabschnitt
- 23a: Teilabschnitt
- 23b: Teilabschnitt
- 24: Kopfabschnitt
- 24a: Verbindungsmittel
- 25: Linksgewinde
- 26: Schlitz
- 27: Längsmittelachse
- 28: Winkel
- 30: Verbindungsabschnitt
- 31: Steg
- 32: Verankerungsteil
- 33: Verankerungsteil
- 34: Gaumennaht
- 35: Gaumen
- 36: Stift
- 37: Halteelement
- 38: Nut
- 39: Hilfsvorrichtung
- 40: Führungsrillenabschnitt
- 41: Führungsrillenabschnitt

## Patentansprüche

1. Schraubgetriebe umfassend
eine erste und eine zweite Schraube (1, 2), die jeweils ein Kopfende (11, 21) und ein gegenüberliegendes Fußende (12, 22) aufweisen, wobei eine jeweilige Schraube (1, 2) im Bereich des Kopfendes (11, 21) mit einem anderen Teil einer das Schraubgetriebe aufweisenden Vorrichtung verbindbar ist, und von denen die erste Schraube (1) einen Gewindeabschnitt (13) mit einem Rechtsgewinde (15) und die zweite Schraube (2) einen Gewindeabschnitt (23) mit einem Linksgewinde (25) aufweist, und
eine gemeinsame Mutter (3), welche eine Durchgangsöffnung (4) aufweist, deren Wandung mit einem ersten Gegengewinde (5) für die erste Schraube (1) und einem zweiten Gegengewinde (6) für die zweite Schraube (2) versehen ist, wobei die erste und zweite Schraube von gegenüberliegenden Seiten der Mutter (3) her in die Gegengewinde (5, 6) der Mutter (3) einschraubbar sind, **dadurch gekennzeichnet, dass**
die Gewindeabschnitte (13, 23) der ersten und zweiten Schraube (1, 2) jeweils mindestens zwei Teilabschnitte (13a, 13b; 23a, 23b) aufweisen, deren Außenflächen Umfangsteile von Kreiszylinder-Mantelflächen bilden, die mit dem Rechts- bzw. Linksgewinde (15, 25) versehen sind, wobei zumindest eine der Schrauben (1, 2) im Bereich ihrer Längsmittelachse (17, 27) einen von ihrem Fußende (12, 22) ausgehenden Schlitz (16, 26) aufweist, der die Teilabschnitte (13a, 13b; 23a, 23b) zumindest über einen Teil ihrer axialen Erstreckung trennt, wobei die mit ihren Fußenden (12, 22) aufeinander zu gerichteten ersten und zweiten Schrauben (1, 2) axial ineinander einsteckbar sind,
dass die ersten und zweiten Gegengewinde (5, 6) der Mutter (3) zumindest über einen Teil ihrer axialen Erstreckungen ineinander liegend in den gleichen axialen Abschnitt der Wandung der Durchgangsöffnung (4) eingebracht sind,
dass die ersten und zweiten Gegengewinde (5, 6) über mindestens zwei Drittel ihrer axialen Erstreckung ineinander liegend in den gleichen axialen Abschnitt der Wandung der Durchgangsöffnung (4) eingebracht sind und dass die ersten und zweiten Schrauben (1, 2) über zumindest zwei Drittel der axialen Erstreckungen der Gewindeabschnitte (13, 23) der ersten und zweiten Schrauben (1, 2) axial ineinander einsteckbar sind.

2. Schraubgetriebe nach Anspruch 1, **dadurch gekennzeichnet, dass** sich die Teilabschnitte (13a, 13b; 23a, 23b) der Gewindeabschnitte (13, 23) der ersten und zweiten axial ineinander eingesteckten Schrauben (1, 2) im Bereich der axialen Überlappung der Teilabschnitte (13a, 13b; 23a, 23b) zu einem vollständigen Kreiszylinder ergänzen.

3. Schraubgetriebe nach Anspruch 1 oder 2, **dadurch gekennzeichnet, dass** nur eine der beiden Schrauben (1, 2) im Bereich ihrer Längsmittelachse (17, 27) einen von ihrem Fußende (12, 22) ausgehenden Schlitz (16, 26) aufweist, der die Teilabschnitte (13a, 13b; 23a, 23b) trennt, und dass die Teilabschnitte (13a, 13b; 23a, 23b) der anderen der beiden Schrauben (1, 2) im Bereich der Längsmittelachse (17, 27) dieser Schraube (1, 2) über ihre gesamte axiale Erstreckung miteinander verbunden sind.

4. Schraubgetriebe nach Anspruch 1 oder 2, **dadurch gekennzeichnet, dass** beide Schrauben (1, 2) im Bereich ihrer Längsmittelachse (17, 27)jeweils einen vom Fußende (12, 22) der jeweiligen Schraube (1, 2) ausgehenden Schlitz (16, 26) aufweisen, wobei der Schlitz (16, 26) die Teilabschnitte (13a, 213b; 23a, 23b) nur über einen Teil der axialen Erstreckung der Teilabschnitte (13a, 13b; 23a, 23b) trennt und/oder ein Stift (36) im von den Schlitzen (16, 26) gebildeten Aufnahmeraum angeordnet ist, der sich sowohl innerhalb des Schlitzes (16) der ersten Schraube (1) als auch innerhalb des Schlitzes (26) der zweiten Schraube (2) erstreckt.

5. Schraubgetriebe nach einem der Ansprüche 1 bis 4, **dadurch gekennzeichnet, dass** eine jeweilige der Schrauben (1, 2) im Bereich ihres Kopfendes (11, 21) einen Kopfabschnitt (14, 24) aufweist, der in axialer Richtung an den Gewindeabschnitt (13, 23) der jeweiligen Schraube (1, 2) anschließt.

6. Schraubgetriebe nach einem der Ansprüche 1 bis 5, **dadurch gekennzeichnet, dass** der Gewindeabschnitt (13, 23) einer jeweiligen der Schrauben (1, 2) genau zwei Teilabschnitte (13a, 13b; 23a, 23b) aufweist, deren Außenflächen, welche Umfangsteile von Kreiszylinder-Mantelflächen bilden, die mit dem Rechts- bzw. Linksgewinde (15, 25) versehen sind, sich jeweils über einen Winkel 18, 28 von 80° bis 100° um die Längsmittelachse (17, 27) erstrecken.

7. Expandervorrichtung zur Gaumennahterweiterung mit einem Schraubgetriebe nach einem der Ansprüche 1 bis 6, dessen erste und zweite Schrauben (1, 2) über Verbindungsteile (30, 31) mit Verankerungsteilen (32, 33) zur Verankerung der Expandervorrichtung beidseitig der Gaumennaht (34) verbunden sind.

8. Kombination eines Schraubgetriebes nach einem der Ansprüche 1 bis 6 mit einer Hilfsvorrichtung zur Erleichterung der Montage des Schraubgetriebes, **dadurch gekennzeichnet, dass** die Hilfsvorrichtung gegenüberliegende Führungsrillenabschnitte (40, 41) aufweist, entlang von welcher an die Kopfenden (11, 21) der Schrauben (1, 2) anschließende Kopfabschnitte (14, 24) der Schrauben (1, 2) verdrehgesichert gleitend geführt sind, wobei sich die Abschnitte der Führungsrille (40) in eine Richtung rechtwinkelig zur Längserstreckung der Schrauben (1, 2) aneinander annähern.

## Claims

1. A screw mechanism, comprising
a first and a second screw (1, 2), which each have a head end (11, 21) and an opposite foot end (12, 22), wherein a respective screw (1, 2) in the region of the head end (11, 21) can be connected to another part of a device which includes the screw mechanism, and of which the first screw (1) has a threaded portion (13) with a right-hand thread (15) and the second screw (2) has a threaded portion (23) with a left-hand thread (25), and
a common nut (3) which has a passage opening (4), the wall of which is provided with a first mating thread (5) for the first screw (1) and a second mating thread (6) for the second screw (2), wherein the first and second screw can be screwed into the mating thread (5, 6) of the nut (3) from opposite sides of the nut (3),
**characterised in that**
the threaded portions (13, 23) of the first and second screw (1, 2) each have at least two partial portions (13a, 13b; 23a, 23b), the outer surfaces of which form peripheral parts of circular-cylinder circumferential surfaces which are provided with the right-hand or left-hand thread (15, 25), at least one of the screws (1, 2) having in the region of its longitudinal centre line (17, 27) a slot (16, 26) starting from its foot end (12, 22), which slot separates the partial portions (13a, 13b; 23a, 23b) at least over a portion of their axial extent, the first and second screws (1, 2) directed towards each other with their foot ends (12, 22) being able to be inserted axially into one another,
**in that** the first and second mating thread (5, 6) of the nut (3), at least over a portion of their axial extents, are introduced into the same axial portion of the wall of the passage opening (4), lying one in another,
**in that** the first and second mating thread (5, 6) are introduced over at least two thirds of their axial extent into the same axial portion of the wall of the passage opening (4), lying one in another, and
**in that** the first and second screws (1, 2) can be inserted axially into one another over at least two thirds of the axial extents of the threaded portions (13, 23) of the first and second screws (1, 2).

2. A screw mechanism according to claim 1, **characterised in that** the partial portions (13a, 13b; 23a, 23b) of the threaded portions (13, 23) of the first and second screws (1, 2) inserted axially into one another, in the region of the axial overlapping of the partial portions (13a, 13b; 23a, 23b), complete a circular cylinder.

3. A screw mechanism according to claim 1 or 2, **characterised in that** only one of the two screws (1, 2) has in the region of its longitudinal centre line (17, 27) a slot (16, 26) starting from its foot end (12, 22), which slot separates the partial portions (13a, 13b; 23a, 23b), and **in that** the partial portions (13a, 13b; 23a, 23b) of the other one of the two screws (1, 2) in the region of the longitudinal centre line (17, 27) of this screw (1, 2) are connected together over their entire axial extent.

4. A screw mechanism according to claim 1 or 2, **characterised in that** both screws (1, 2) in the region of their longitudinal centre lines (17, 27) each have a slot (16, 26) starting from the foot end (12, 22) of the respective screw (1, 2), the slot (16, 26) separating the partial portions (13a, 213b [sic]; 23a, 23b) only over a part of the axial extent of the partial portions (13a, 13b; 23a, 23b) and/or a pin (36) being arranged in the receiving space formed by the slots (16, 26), which receiving space extends both within the slot (16) of the first screw (1) and within the slot (26) of the second screw (2).

5. A screw mechanism according to one of claims 1 to 4, **characterised in that** a respective one of the screws (1, 2) has in the region of its head end (11, 21) a head portion (14, 24) which adjoins the threaded portion (13, 23) of the respective screw (1, 2) in the axial direction.

6. A screw mechanism according to one of claims 1 to 5, **characterised in that** the threaded portion (13, 23) of a respective one of the screws (1, 2) has precisely two partial portions (13a, 13b; 23a, 23b), the outer surfaces of which, which form peripheral parts of circular-cylinder circumferential surfaces which are provided with the right-hand or left-hand thread (15, 25), each extend about the longitudinal centre line (17, 27) through an angle 18, 28 of 80° to 100°.

7. An expander device for palatal expansion, having a screw mechanism according to one of claims 1 to 6, the first and second screws (1, 2) of which are connected by way of connecting parts (30, 31) to anchoring parts (32, 33) for anchoring the expander device on either side of the midpalatal suture (34).

8. A combination of a screw mechanism according to one of claims 1 to 6 with an auxiliary device for facilitating the assembly of the screw mechanism, **characterised in that** the auxiliary device has opposing guide groove portions (40, 41), along which head portions (14, 24) of the screws (1, 2) which adjoin the head ends (11, 21) of the screws (1, 2) are guided so as to slide in a manner secured against twisting, the portions of the guide groove (40) converging in a direction at right-angles to the longitudinal extent of the screws (1, 2).

## Revendications

1. Engrenage hélicoïdal comprenant
une première et une deuxième vis (1, 2) qui présentent chacune une extrémité de tête (11, 21) et une extrémité de pied (12, 22) opposée, chaque vis (1, 2) pouvant être reliée au niveau de l'extrémité de tête (11, 21) à une autre partie d'un dispositif présentant l'engrenage hélicoïdal, et dont la première vis (1) présente une section filetée (13) avec un filetage à droite (15) et la deuxième vis (2) une section filetée (23) avec un filetage à gauche (25), et
un écrou commun (3) qui présente une ouverture de passage (4) dont la paroi est pourvue d'un premier contre-filet (5) pour la première vis (1) et d'un deuxième contre-filet (6) pour la deuxième vis (2), la première et la deuxième vis pouvant être vissées dans les contre-filets (5, 6) de l'écrou (3) à partir de côtés opposés de l'écrou (3),
**caractérisé**
**en ce que** les sections filetées (13, 23) de la première et de la deuxième vis (1, 2) comportent chacune au moins deux sections partielles (13a, 13b ; 23a, 23b), dont les surfaces extérieures forment des parties périphériques de surfaces d'enveloppe de cylindre circulaire, qui sont munies du filetage à droite ou à gauche. (15, 25), au moins l'une des vis (1, 2) présentant, au niveau de son axe médian longitudinal (17, 27), une fente (16, 26) qui part de son extrémité de pied (12, 22) et qui sépare les sections partielles (13a, 13b ; 23a, 23b) au moins sur une partie de leur extension axiale, les première et deuxième vis (1, 2) dirigées l'une vers l'autre par leurs extrémités de pied (12, 22) pouvant être emboîtées axialement l'une dans l'autre,
**en ce que** les premier et deuxième contre-filets (5, 6) de l'écrou (3) sont insérés dans la même section axiale de la paroi de l'ouverture de passage (4) en étant imbriqués l'un dans l'autre au moins sur une partie de leurs extensions axiales,
**en ce que** les premier et deuxième contre-filets (5, 6) sont insérés sur au moins deux tiers de leur extension axiale, imbriqués l'un dans l'autre, dans la même section axiale de la paroi de l'ouverture de passage (4), et
**en ce que** les première et deuxième vis (1, 2) peuvent être imbriquées axialement l'une dans l'autre sur au moins deux tiers des extensions axiales des sections filetées (13, 23) des première et deuxième vis (1, 2).

2. Engrenage hélicoïdal selon la revendication 1, **caractérisé en ce que** les sections partielles (13a, 13b ; 23a, 23b) des sections filetées (13, 23) des première et deuxième vis (1, 2) imbriquées axialement l'une dans l'autre se complètent au niveau du chevauchement axial des sections partielles (13a, 13b ; 23a, 23b) pour former un cylindre circulaire complet.

3. Engrenage hélicoïdal selon la revendication 1 ou 2, **caractérisé en ce que** seule l'une des deux vis (1, 2) présente une fente (16, 26) au niveau de son axe médian longitudinal (17, 27), laquelle fente part de son extrémité de pied (12, 22) et sépare les sections partielles (13a, 13b ; 23a, 23b), et **en ce que** les sections partielles (13a, 13b ; 23a, 23b) de l'autre des deux vis (1, 2) sont reliées ensemble au niveau de l'axe médian longitudinal (17, 27) de cette vis (1, 2) sur toute leur étendue axiale.

4. Engrenage hélicoïdal selon la revendication 1 ou 2, **caractérisée en ce que** les deux vis (1, 2) présentent chacune, au niveau de leur axe médian longitudinal (17, 27), une fente (16, 26) partant de l'extrémité de pied (12, 22) de la vis correspondante (1, 2), la fente (16, 26) ne séparant les sections partielles (13a, 213b ; 23a, 23b) que sur une partie de l'extension axiale des sections partielles (13a, 13b ; 23a, 23b), et/ou une tige (36) est disposée dans l'espace de réception formé par les fentes (16, 26) et s'étend à la fois à l'intérieur de la fente (16) de la première vis (1) et à l'intérieur de la fente (26) de la deuxième vis (2).

5. Engrenage hélicoïdal selon l'une des revendications 1 à 4, **caractérisé en ce que** chacune des vis (1, 2) présente, au niveau de son extrémité de tête (11, 21), une portion de tête (14, 24) qui prolonge, dans la direction axiale, à la section filetée (13, 23) de la vis correspondante (1, 2).

6. Engrenage hélicoïdal selon l'une des revendications 1 à 5, **caractérisé en ce que** la section filetée (13, 23) de chacune des vis (1, 2) présente exactement deux sections partielles (13a, 13b ; 23a, 23b) dont les surfaces extérieures, qui forment des parties périphériques de surfaces d'enveloppe de cylindre circulaire pourvues du filetage à droite ou à gauche (15, 25), s'étendent chacune sur un angle (18, 28) de 80° à 100° autour de l'axe médian longitudinal (17, 27).

7. Dispositif d'expansion pour l'élargissement de la suture palatine avec un engrenage hélicoïdal selon l'une des revendications 1 à 6, dont les première et deuxième vis (1, 2) sont reliées par des pièces de liaison (30, 31) à des pièces d'ancrage (32, 33) pour ancrer le dispositif d'expansion de part et d'autre de la suture palatine (34).

8. Combinaison d'un engrenage hélicoïdal selon l'une des revendications 1 à 6 avec un dispositif auxiliaire pour faciliter le montage de l'engrenage hélicoïdal, **caractérisée en ce que** le dispositif auxiliaire présente des sections de rainures de guidage (40, 41) opposées le long desquelles sont guidées de manière coulissante et bloquées en rotation des portions de tête (14, 24) des vis (1, 2) prolongeant les extrémités de tête (11, 21) des vis (1,2), les sections de la rainure de guidage (40) se rapprochant l'une de l'autre dans une direction perpendiculaire à l'extension longitudinale des vis (1, 2).
